# FASCICULE DE BREVET EUROPEEN

(11) **EP 1 756 193 B1**
(45) Date de publication et mention de la délivrance du brevet: **11.11.2009**
(21) Numéro de dépôt: 05746645.0
(22) Date de dépôt: 20.04.2005
(51) Int. Cl.: C08G 61/12, C07D 333/18, C08G 61/02

(54) **COMPOSES MONO-, OLIGO ET POLYMERES CONJUGUES, ET CELLULES PHOTOVOLTAIQUES LES CONTENANT**
MONOMER-, OLIGOMER- UND PI-KONJUGIERTE POLYMERVERBINDUNGEN UND PHOTOVOLTAISCHE ZELLEN, WELCHE DIESE ENTHALTEN
MONOMER, OLIGOMER AND PI-CONJUGATED POLYMER COMPOUNDS, AND PHOTOVOLTAIC CELLS CONTAINING SAME

(30) Priorité: 21.04.2004 FR 0404213
(43) Date de publication de la demande: 28.02.2007
(73) Titulaire: COMMISSARIAT A L'ENERGIE ATOMIQUE, 75015 Paris (FR)
(72) Inventeur: DEMADRILLE, Renaud, F-38120 SAINT EGREVE (FR); PRON, Adam, F-38120 SAINT EGREVE (FR); FIRON, Muriel, F-91250 EGLY (FR); LEROY, Jocelyne, F-91470 BOULLAY LES TROUX (FR)
(74) Mandataire: Poulin, Gérard
(86) Numéro de dépôt international: PCT/FR2005/050264
(87) Numéro de publication internationale: WO 2005/105884

(56) Documents cités:
- EP-A- 1 327 646
- EP-A1- 1 524 286
- EP-A1- 1 542 294
- WO-A-03/078498
- WO-A1-2005/092947
- US-A- 4 390 608
- US-A1- 2003 062 536
- EL HADJ ELANDALOUSSI ET AL.: "Effect of Chain Extension on the Electrochemical and Electronic Properties of pi-Conjugated Soluble Thienylenevinylene Oligomers" JOURNAL OF THE AMERICAN CHEMICAL SOCIETY, vol. 119, 1997, pages 10774-10784, XP002304424 cité dans la demande
- GREVIN ET AL.: "Multiscale Scanning Tunneling Miscroscopy Study of Self-Assembly Phenomena in Two-Dimentional Polycrystals of PI-Conjugated Polymers: The case of Regioregular Poly(dioctylbithiophene-alt-fluorenone)" ADVANCED MATERIAL, vol. 16, 17 décembre 2004 (2004-12-17), pages 2087-2092, Weinheim

## Description

### DOMAINE TECHNIQUE

L'invention concerne de nouveaux composés mono, oligo et polymères π-conjugués, en particulier l'invention concerne des nouveaux composés comprenant au moins un motif avec une structure fluorénone ou un dérivé de celle-ci, et éventuellement des motifs thiénylène-vinylènes.

L'invention concerne également l'utilisation de ces composés dans des dispositifs opto-électroniques, et les cellules photovoltaïques contenant ces composés.

Le domaine technique de l'invention peut être défini comme celui des semiconducteurs organiques et notamment des hétérojonctions.

Un grand nombre d'applications des semiconducteurs organiques concerne leur utilisation dans des systèmes électroniques tels que les diodes électroluminescentes, les transistors à effets de champ ou encore les cellules photovoltaïques. Cette dernière application des semiconducteurs organiques est basée sur la capacité de certains polymères et oligomères π-conjugués, ou encore de petites molécules π-conjuguées, à convertir l'énergie lumineuse en énergie électrique.

Lorsqu'on constitue une jonction composée de deux semiconducteurs de nature différente parmi lesquels au moins un est organique, on définit ainsi une hétérojonction.

Les mélanges en hétérojonction anisotype, c'est-à-dire entre un semiconducteur organique de type p et un semiconducteur organique ou inorganique de type n connaissent depuis quelques années de nombreuses applications dans le domaine de l'électronique plastique, par exemple dans les diodes électroluminescentes et les cellules photovoltaïques. Généralement, dans celles-ci, le polymère, oligomère π-conjugué, où la petite molécule π-conjuguée citée plus haut est mise en présence d'un accepteur de type n tel que le fullerène ou un dérivé de celui-ci, et il (elle) joue le rôle de donneur de type p.

Sous irradiation lumineuse, une paire électron-trou est créée (exciton) sur le monomère, oligomère, ou polymère π-conjugué. Cet exciton est dissocié par capture de l'électron par l'accepteur. Ces charges sont collectées aux électrodes et génèrent un courant.

Des hétérojonctions dans le domaine du photovoltaïque sont décrites notamment dans le document WO-A-03/078998 qui concerne des polymères conjugués dérivés du PolyPhenyleneVinylene (PPV). Ces polymères électriquement conducteurs sont utilisés notamment dans les couches actives des cellules photovoltaïques.

Un des problèmes majeurs de ces cellules concerne l'absorption de la lumière ; en effet, afin d'être efficace le spectre d'absorption du polymère ou de l'oligomère doit se rapprocher le plus possible du spectre d'émission solaire. Plus l'absorption de la lumière est efficace, plus la possibilité de créer des excitons est importante. Dans le cas du PPV et de ses dérivés, le domaine d'absorption ne dépasse pas 650 nm à l'état solide.

Pour résoudre ce problème, on a cherché à augmenter le domaine d'absorption de la couche active en utilisant des polymères à faible "gap", par exemple à base de thiénylpyrrole et de benzodithiazole comme cela est décrit dans le document "Molecular Crystals Liquid Crystals", 385, 213-220, 2002, ou bien en utilisant des dérivés Méthano[70]fullerène qui absorbent plus loin dans le visible comme cela est mentionné dans l'article "Efficient Methano[70]fullerene/MDMO-PPV Bulk Heterojunction Photovoltaic Cells" de M.M. WIENK et al., Angew. Chem. Int. Ed. 2003, 42, 3371-3375.

Une autre famille de polymères a été récemment étudiée, il s'agit de la famille des poly(alkythiophène). Ainsi, l'article de Applied Physics Letters, vol. 81, Num. 20, P.3885 (2002), décrit-il des hétérojonctions comprenant un mélange de poly-3(hexylthiophène) et d'un méthanofullerène.

Les poly(alkylthiophènes) ont également été étudiés en mélange avec des nanocristaux inorganiques de semiconducteurs du type CdSe dans l'article "Hybrid Nanorod-Polymer Solar Cells" de W.U. HUYNH et al. dans Science, vol. 295, March 2002.

Dans le cas de l'utilisation des poly(alkylthiophène), seuls les polymères régioréguliers sont utilisés pour des raisons de mobilité des charges. En effet, le transport des charges est un autre point critique dans ces dispositifs.

La régiorégularité favorise l'autoorganisation et permet d'augmenter le degré de cristallinité des matériaux, ce qui est favorable pour le transport électrique. Il est en outre à noter que les poly(alkylthiophène) régioréguliers présentent également des domaines d'absorption plus étendus que les poly(alkylthiophène) statistiques.

Par ailleurs, les dérivés du fluorène sont également des matériaux connus pour leurs bonnes propriétés optoélectroniques. Ainsi, le document EP-A2-1 282 258 décrit-il l'utilisation de composés mono-, oligo-, et polyalkylidènefluorènes comme transporteurs de charges dans les dispositifs électriques tels que les transistors à effet de champ, les diodes électroluminescentes, les cellules photovoltaïques et les capteurs.

Les polymères semiconducteurs organiques décrits dans les documents de l'art antérieur présentent un domaine d'absorption qui ne se rapproche pas suffisamment près du spectre d'émission solaire, qui ne s'étend pas suffisamment dans le domaine visible. En outre, la génération, et la dissociation des excitons est insuffisante, pouvant être à l'origine d'une mobilité des porteurs de charge et d'un transport aux électrodes trop faibles.

Il existe donc un besoin pour des composés, un matériau, et en particulier pour un monomère, oligomère, ou polymère semiconducteur organique dont le domaine d'absorption se situe de manière très étendue dans le spectre d'émission solaire, qui génère une importante quantité d'excitons, et en tout cas plus d'excitons que dans les composés, par exemple les polymères de l'art antérieur.

Le but de la présente invention est de fournir un composé organique semiconducteur en particulier pour les cellules photovoltaïques qui réponde entre autres à ces besoins.

Le but de la présente invention est encore de fournir un composé organique semiconducteur, en particulier pour les cellules photovoltaïques qui ne présente pas les inconvénients, défauts, limitations, et désavantages des composés de l'art antérieur et qui résolve les problèmes de l'art antérieur.

Ce but et d'autres encore, sont atteints, conformément à l'invention par des composés monomères, oligomères et polymères répondant à la formule (I) suivante : 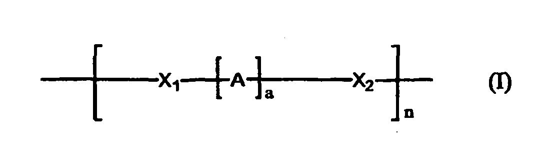 dans laquelle
- A représente un groupe polycyclique, éventuellement substitué par un ou plusieurs groupements R identiques ou différents choisis parmi les radicaux alkyle, ledit groupe polycyclique comprenant au moins deux cycles choisis parmi les cycles aromatiques carbonés et/ou les hétérocycles aromatiques, et comprenant au moins un groupe susceptible de permettre le rattachement d'un groupe chromophore,
- X₁ et X₂ identiques ou différents, représentent chacun indépendamment un groupe de formule (II) : 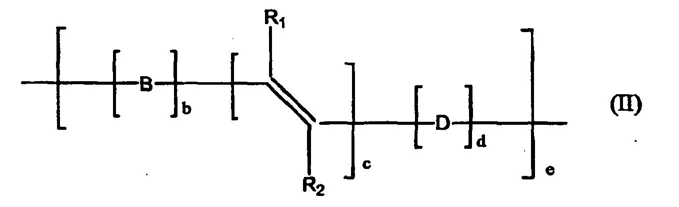 dans laquelle
   - B et D, identiques ou différents représentent chacun indépendamment un cycle aromatique carboné ou un hétérocycle aromatique éventuellement substitué par un ou plusieurs groupements R ;
   - R₁ et R₂, identiques ou différents, représentent chacun indépendamment un groupement choisi parmi l'atome d'hydrogène, les groupements R, les groupements cyano, nitro, les atomes d'halogène et les atomes de deutérium ;
   - b et d sont des nombres entiers de 0 à 100, de préférence de 0 à 12, incluant toutes les valeurs entre 0 et 12 ;
   - c est un nombre entier de 0 à 20, de préférence de 0 à 5, incluant toutes les valeurs entre 0 et 5 ;
   - e est un nombre entier de 0 à 100, de préférence de 0 à 10, incluant toutes les valeurs entre 0 et 10 ;
   - X₁ et/ou X₂ sont éventuellement substitués par un ou plusieurs groupes de formule (II) identiques ou différents du groupe X₁ ou X₂ substitué ;
- n est un nombre entier de 1 à 1000, de préférence de 1 à 200, incluant toutes les valeurs entre 1 et 200 ;
- a est un nombre entier de 1 à 100, de préférence de 1 à 5, incluant toutes les valeurs entre 1 et 5 ; ,
- un ou plusieurs des atomes d'hydrogène de la formule (I) pouvent être remplacés par un atome de deutérium ou de fluor.

Le composé selon l'invention peut être un monomère (n=1).

Ce monomère peut être symétrique ou non symétrique, mais de préférence il est symétrique ; en effet à partir de ces monomères symétriques des oligomères et polymères alternés, régioréguliers, sans défauts de couplage, peuvent être obtenus par homopolymérisation.

Par "symétrique", on entend généralement que X₁ est identique à X₂ dans la formule (I).

Avantageusement, le groupe susceptible de permettre le rattachement d'un groupe chromophore est un groupe 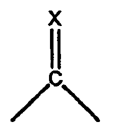 où X représente 0, S, Se ou N-R₃, R₃ étant choisi parmi les groupes issus de la réaction d'un groupe carbonyle (X=O) avec une amine pour former une liaison imine.

Le groupement carbonyle (X=O) peut être modifié par réaction avec n'importe quelle amine pour former une liaison imine, de préférence cette amine est choisie parmi l'aniline et les dérivés de celle-ci, les oligomères de l'aniline, et les dérivés de ceux-ci, et de préférence encore cette amine est le tétramère de l'aniline sous la forme éméraldine base.

Avantageusement, dans la formule (I), le groupe polycyclique du groupe A est un groupe polycyclique condensé plutôt qu'un groupe polycyclique comprenant plusieurs cycles liés par des liaisons simples ou des groupes de liaison.

Avantageusement, dans la formule (I), le groupe A est choisi parmi les groupements fluorènone, truxénone, indénofluorénone, benzofluorénone, dibenzofluorénone, indénofluorénedione, cyclopentafluorénone, cyclopentafluorénedione, thiopyranone, phénanthrénone, cyclopentadithiophénone, et les groupements dérivés de ceux-ci (dérivés des groupements énumérés ci-dessous) ; les groupements dérivés de la xanthone de formule 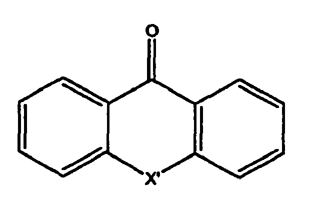 où X' représente un radical -CO-, -S-, -Se-, -NH-, -CH₂-, -CH₂=CH₂- ; les groupements dérivés de la 4-pipéridone comprenant au moins deux cycles aromatiques carbonés et/ou hétérocycles aromatiques condensés, ces groupements étant éventuellement substitués par un ou plusieurs groupements R et/ou éventuellement condensés avec un ou plusieurs cycles aromatiques condensés et/ou hétérocycles aromatiques.

Par ailleurs, les oligomères et polymères selon l'invention peuvent contenir des groupes A de même nature ou de nature différente.

Il s'agit de préférence d'oligomères et de polymères contenant de préférence un seul type de groupe A, régioréguliers, sans défauts de couplage, qui sont obtenus à partir des monomères symétriques ci-dessus.

Les polymères régioréguliers peuvent être obtenus sans défauts de couplage même avec une masse moléculaire élevée à savoir généralement de 2000 à 200 000 Da.

De préférence dans la formule (II), B et D représentent un groupe thiophène, de préférence un groupe thièn-2,5-diyle.

De préférence, B et D représentent tous deux un tel groupe.

De préférence B et D, en particulier lorsqu'ils représentent un thiophène tel que défini ci-dessus sont substitués par un radical alkyle de 1 à 10C, tel que le radical n-octyle.

En effet, il s'est avéré que lorsque les unités B et D de type thiénylène, par exemple, portent des groupes alkyles, la solubilité du système est augmentée, ce qui autorise une mise en oeuvre par voie liquide.

Il est à noter que le rattachement des cycles B et D et la position du ou des substituants R et R₁ et R₂ est choisie de préférence à obtenir une symétrie de la molécule dans le cas d'un monomère, une telle symétrie du monomère conduisant ensuite à des polymères, régioréguliers, alternés.

De préférence, le groupe A est un groupement fluorénone ou dérivé de celle-ci et il répond à la formule suivante : 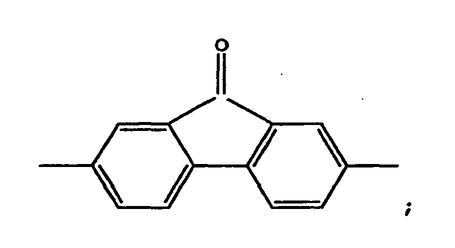 et X₁ et X₂, identiques ou différents répondent à la formule suivante : 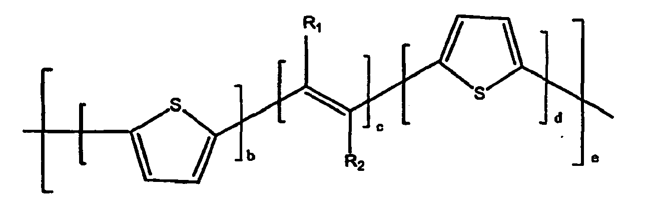 le groupe A et les groupes thiényle pouvant être éventuellement substitués par un ou plusieurs groupements R.

Un composé selon l'invention particulièrement préféré répond à la formule suivante :

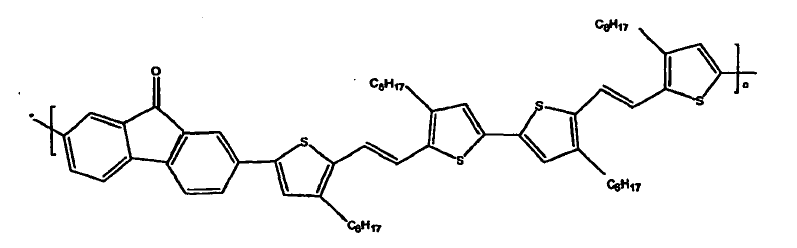

Ce composé est le 2,7-bis(5-[(E)-1,2-bis(3-octylthièn-2-yl)éthylène])-fluorèn-9-one (TVF) si n=1 (monomère) ; ou le poly(2,7-bis(5-[(E)-1,2-bis(3-octylthièn-2-yl) éthylène])-fluorèn-9-one)) (poly(TVF)) si n est supérieur à 1 (oligomère ou polymère).

Dans les formules (I) et (II), ci-dessus, le terme alkyle utilisé pour les radicaux alkyle, ainsi que pour les groupements comportant une partie alkyle, signifie, sauf indication différente, une chaîne carbonée, linéaire ou ramifiée, comportant de 1 à 30 atomes de carbone, de préférence de 1 à 10, mieux de 1 à 8, pouvant comporter une ou plusieurs doubles liaisons ou triples liaisons carbone-carbone, et/ou pouvant être portée et/ou interrompue par un ou plusieurs atomes d'oxygène, de soufre, de silicium, ou d'azote, et/ou pouvant être substituée par un ou plusieurs groupes choisis parmi les atomes d'halogène, tel le chlore, le brome, l'iode et le fluor ; les hétérocycles ; les radicaux aryle ; hydroxyle ; alcoxy ; amino ; acyle ; carboxamido ; -CO₂H ; -SO₃H ; -PO₃H₂ ; -PO₄H₂ ; -NHSO₃H ; sulfonamide ; monoalkylamino ; trialkylammonium ; ou bien encore par un radical dialkylamino, dans lequel les deux groupements alkyle peuvent former, conjointement avec l'atome d'azote dudit groupement dialkyl(C₁-C₄)amino, auquel ils sont liés, un cycle pouvant être interrompu par un ou plusieurs atomes d'azote, d'oxygène ou de soufre.

De même, selon l'invention, le terme alcoxy utilisé pour les radicaux alcoxy ainsi que pour les groupements comportant une partie alcoxy, signifie, sauf indication différente, une chaîne O-alkyle, le terme alkyle ayant la signification indiquée ci-dessus. Les radicaux alcoxy des groupes alcoxycarbonyle ont, de préférence, de 1 à 4 atomes de carbone. Les groupes acyle ont, de préférence, de 2 à 4 atomes de carbone.

Selon l'invention, on entend par hétérocycle, un cycle aromatique lorsque cela est précisé (ou non aromatique) contenant 5, 6 ou 7 sommets, et de 1 à 3 hétéroatomes choisis parmi les atomes d'azote, de soufre et d'oxygène. Ces hétérocycles peuvent être condensés sur d'autres hétérocycles, ou sur d'autres cycles notamment aromatiques tels qu'un groupement phényle. Ces hétérocycles peuvent, en outre, être quaternisés par un radical alkyle. Les termes alkyle et alcoxy ont les significations indiqués ci-dessus.

Parmi, les hétérocycles, et notamment ceux de B et D, on peut notamment citer à titre d'exemple les cycles : thiophène, benzothiophène, furane, benzofurane, indole, indoline, carbazole, pyridine, déhydroquinoléine, chromone, julodinine, thiadiazole, triazole, isoxazole, oxazole, thiazole, isothiazole, imidazole, pyrazole, triazine, thiazine, pyrazine, pyridazine, pyrimidine, pyridine, diazépine, oxazépine, benzotriazole, benzoxazole, benzimidazole, benzothiazole, morpholine, pipéridine, pipérazine, azétidine, pyrrolidine, aziridine.

Selon l'invention, on entend par cycle aromatique carboné, sauf spécifié autrement, un radical aryle en C₆ à C₃₀ pouvant être substitué par un ou plusieurs radicaux alkyle ; alcoxy ; acyle ; cyano ; carboxamido ; -CO₂H ; -SO₃H ; -PO₃H₂ ; -PO₄H₂ ; hydroxyle ; amino ; monoalkyl(C₁-C₄)amino ; ou dialkyl(C₁-C₄)amino ; dans lequel les deux groupements alkyle peuvent former, conjointement avec l'atome d'azote dudit groupement dialkyl(C₁-C₄)amino. De préférence, le groupe aryle est un groupe phényle ou un groupe naphtyle pouvant être substitué comme indiqué ci-dessus.

L'invention concerne également l'utilisation du composé tel que décrit ci-dessus dans un dispositif optoélectronique tel qu'une cellule photovoltaïque, un transistor à effet de champ ou un capteur électrochimique. L'invention a trait aussi à une cellule photovoltaïque comprenant une couche active comprenant un donneur d'électrons constitué par le composé tel que décrit ci-dessus et un accepteur d'électrons.

Ledit accepteur d'électrons est choisi généralement parmi les accepteurs organiques tels que le PCBM (1-(3-méthoxycarbonyl)-propyl-1-phényl-[6,6]-C61), les dérivés du C₆₀, les dérivés du C₇₀, les nanotubes de carbone, le pérylène, le tétracyanoquinodiméthane (TCNQ) et les quinoxalines ; et les accepteurs inorganiques tels que les nanocristaux de semiconducteurs, enrobés ou non par une couche organique.

Les dérivés du fluorène sont des composés connus pour leurs bonnes propriétés optoélectroniques comme cela est décrit par exemple dans le document EP-A2-1 284 258 déjà cité plus haut.

Ce document ne décrit toutefois aucun monomère, oligomère, ou polymère de fluorènone.

Les polymères contenant des fluorènones ont été très peu étudiés. Les documents traitant de ces composés dans le cadre de dispositifs optoélectroniques indiquent que les fluorènones sont considérés comme des produits de dégradation des polyfluorènes. La présence de fluorènone n'est pas en fait souhaitable dans les dispositifs optoélectroniques, car les fluorènones modifient de manière incontrôlable les couleurs d'émission des diodes électroluminescentes comme cela est mentionné dans le document Advanced Functional Materials, 13(4), 325-330, 2003.

Les matériaux contenant des motifs fluorènones n'ont jamais été mentionnés dans le cadre d'une utilisation dans les cellules photovoltaïques ni dans les transistors à effet de champ.

La préparation des composés de fluorènone selon l'invention en vue de leur mise en oeuvre dans des cellules photovoltaïques et des transistors à effet de champ va donc à l'encontre d'un préjugé largement répandu dans ce domaine de la technique et triomphe de ce préjugé.

Ainsi, le document US-2003/00 81432 A1 concerne un procédé de préparation de polymères comprenant des structures cyclopentanones, par exemple d'un polymère poly(9-fluorénone) et l'utilisation de ces polymères dans des diodes électroluminescentes (DEL). Ce document est antérieur au document "Advanced Functional Materials" de 2003 cité plus haut qui met l'accent sur les inconvénients graves que ces polymères provoquent dans les DEL. En outre, ce document ne mentionne ni ne suggère que les polymères qui y sont préparés puissent être utilisés dans les cellules photovoltaïques et les transistors à effet de champ.

Il est clair que les polymères préparés dans le document pré-cité ne conviennent en aucun cas pour une utilisation dans les transistors à effet de champ et les cellules photovoltaïques et que leurs propriétés en matière de domaine d'absorption, de génération d'excitons, de mobilité des charges et de solubilité sont insuffisantes et ne sont pas adaptées à un usage dans ces dispositifs.

Les matériaux selon l'invention qu'ils s'agissent de monomères, d'oligomères ou de polymères sont fondamentalement définis par une structure, une architecture π-conjuguée, qui comporte plusieurs types de chromophore, à savoir généralement d'une part les chromophores présents dans les groupes A, qui sont de préférence des fluorénones ou des dérivés de celle-ci, et d'autre part les chromophores présents dans les groupes X₁ et/ou X₂ qui sont de préférence des motifs thiénylène-vinylène.

La présence de plusieurs types de chromophores étend le spectre d'absorption des composés de l'invention dans la partie visible du spectre, améliore aussi la collecte des photons, et par voie de conséquence favorise aussi la génération des excitons et permet de générer plus d'excitons que dans les composés de l'art antérieur qui ne possèdent pas la structure particulière des composés selon l'invention.

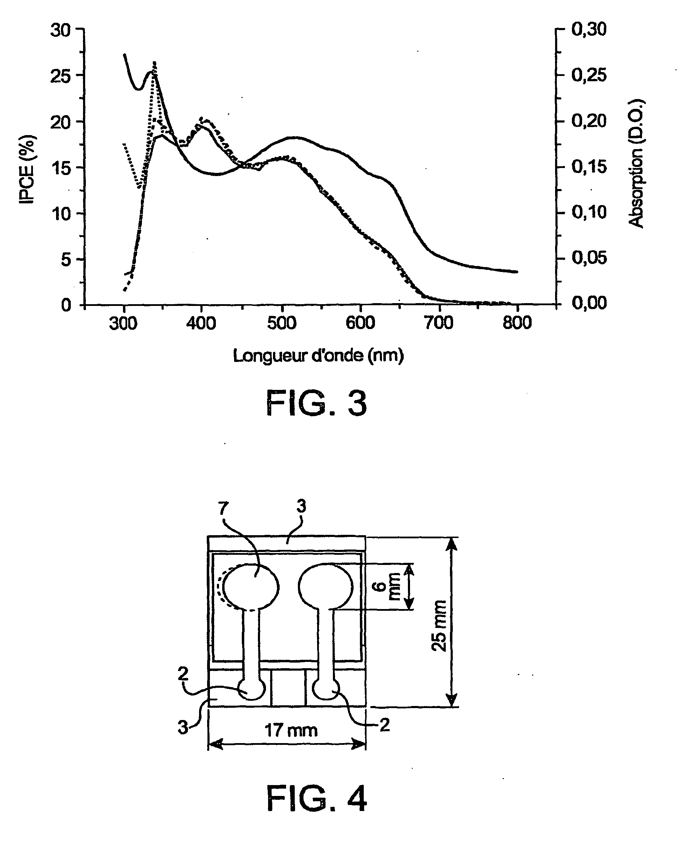

La structure des composés selon l'invention s'est par ailleurs avérée très efficaces dans le cadre de la conversion des photons en électrons comme cela est montré sur la figure 3.

En ce qui concerne les polymères selon l'invention, leur architecture macromoléculaire π-conjuguée est de préférence régiochimiquement bien définie, à régiorégularité contrôlée, et la plus plane possible. Cette approche permet d'obtenir des polymères dont l'autoorganisation est favorisée et la cristallinité est optimisée. Par voie de conséquence, la mobilité des charges dans les matériaux de l'invention est augmentée.

Les monomères, oligomères, et polymères selon l'invention ne présentent pas les inconvénients des composés de l'art antérieur et apportent une solution aux problèmes des composés de l'art antérieur.

En outre, en plus de répondre aux problèmes posés, les composés selon l'invention du fait de leur structure particulière possèdent une grande adaptabilité permettant d'affiner les propriétés recherchées.

En particulier, les composés selon l'invention comportent dans le groupe A au moins un groupe susceptible de permettre le rattachement d'un groupe chromophore (groupe chromophore supplémentaire qui vient s'ajouter aux groupes chromophores déjà présents dans les groupes A, X₁ et X₂) .

Ce groupe permettant le rattachement d'un chromophore, tel qu'un groupe carbonyle, dont sont pourvus les composés selon l'invention permet d'introduire en position latérale, d'autres chromophores. En effet, ce groupe tel qu'un groupe carbonyle, est suffisamment réactif pour permettre une telle introduction, mais également suffisamment stable pour éviter une dégradation pendant l'étape de polymérisation.

En d'autres termes, les composés selon l'invention, en particulier les polymères selon l'invention, se distinguent fondamentalement de par leur structure des composés décrits dans les documents de l'art antérieur représentés par exemple par le document WO-A1-03/078 498.

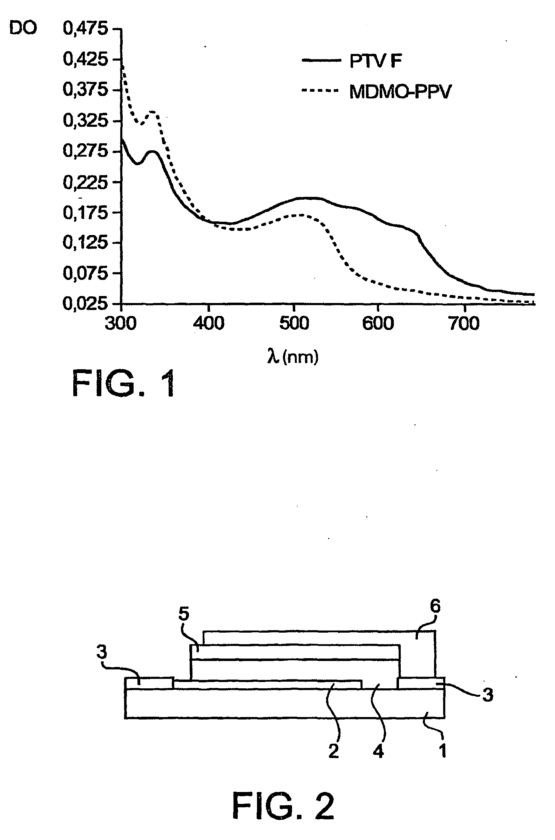

Les polymères selon l'invention présentent un domaine d'absorption plus étendu dans la partie visible du spectre solaire, dû à la présence de plusieurs chromophores et permettent donc de collecter plus de photons dans cette zone. Par voie de conséquence, les composés selon l'invention permettent de générer plus d'excitons (voir figure 1).

Cela ressort clairement de la figure 1 où l'on compare les spectres à l'état solide avec un film de même épaisseur déposé sur du verre d'un mélange PCBM/MDMO-PPV de l'art antérieur, non conforme à l'invention et d'un mélange PCBM/PTVF qui met en jeu un polymère (PTVF) selon l'invention.

Les monomètres de l'invention tels que le TVF se distinguent également de l'art antérieur car ils peuvent être mis en oeuvre en solution comme les polymères.

Dans l'art antérieur, les petites molécules organiques utilisées dans les cellules photovoltaïques sont toutes mises en oeuvre par sublimation comme cela est décrit par exemple dans le document Advanced Materials, 2003, 15, 22.

L'invention sera mieux comprise à la lecture de la description qui va suivre, donnée à titre illustratif et non limitatif, en référence aux dessins joints dans lesquels :
- la figure 1 est un graphique qui représente les spectres à l'état solide pour des films de même épaisseur déposés sur du verre, respectivement d'un mélange PCBM/MDMO-PPV non conforme à l'invention (courbe en traits pointillés) et d'un mélange PCBM/PTVF conforme à l'invention (courbe en trait plein).

En ordonnée est portée la densité optique (D.O.) et en abscisse est portée la longueur d'onde d'absorption λ (en nm).
- La figure 2 est une vue schématique en coupe verticale d'une cellule photovoltaïque présentant une couche active pouvant comporter les composés selon l'invention.
- La figure 3 est un graphique qui donne le spectre d'action, à savoir le rendement de conversion en fonction de la longueur d'onde d'une cellule à base de PTVF (3 courbes en traits pointillés différents pour des conditions opératoires différentes).

Sur ce même graphique, est porté le spectre d'absorption de la couche active (courbe en trait plein).

En abscisse est portée la longueur d'onde λ (en nm), et en ordonnée est porté l'IPCE (%) (à gauche) et la densité optique D.O., absorption (à droite).
- La figure 4 est une vue schématique prise du dessus d'une cellule photovoltaïque.
- La figure 5 est un graphique qui représente la courbe I(V) d'une cellule contenant un mélange PCBM/PTVF selon l'invention dans la couche active.

En abscisse est portée la tension V (volts) et en ordonnée est porté l'intensité I (mA).

La description qui suit est faite essentiellement en référence à la préparation et à la mise en oeuvre d'un monomère symétrique qui est le TVF, à savoir le 2,7-bis(5-[(E)-1,2-bis(3-octylthièn-2-yl)éthylène])-fluorén-9-one, et à la préparation et à la mise en oeuvre d'un polymère alterné régiorégulier sans défaut de PTVF à partir de ce monomère précurseur, mais il est bien évident que cette description peut s'appliquer à tous les monomères oligomères et polymères de formule (I) selon l'invention, moyennant les adaptations nécessaires des procédés et des moyens de mise en oeuvre qui peuvent être facilement réalisés par l'homme du métier dans ce domaine de la technique.

L'approche synthétique pour accéder aux monomères symétriques selon l'invention, de formule (I) peut être décrite de la manière suivante pour ce qui concerne le TVF :
Dans un premier temps :
   Un dérivé thiénylène vinylène tel que le (TV) (3)-1,2-bis(3-octylthièn-2-yl)éthylène est préparé selon une méthode décrite dans la publication J. Am. Chem. Soc., 1997, V. 119, p 10774. Ce dérivé est fonctionnalisé par un ester boronique ou un groupement stanylé d'après des procédures connues telles que celles décrites par exemple dans Org. Letters, 2003, V. 5, 11, 1879-1882 et dans Synth. Metals, 1998, 93, 123-126. Ces groupements fonctionnels sont connus pour être efficaces dans les couplages catalysés au palladium.

Les dérivés ainsi fonctionnalisés peuvent être alors couplés sur la 2,7-dibromofluorénone qui est un produit commercial en utilisant les conditions de couplage de Stille ou de Suzuki bien connues de l'homme du métier.

On obtient ainsi un monomère symétrique tel que le TVF qui, peut alors si on le souhaite, être homopolymérisé par différentes méthodes.

Une première méthode est le couplage oxydant électrochimique ou chimique par exemple en utilisant le chlorure ferrique (FeCl₃) comme cela est décrit par exemple Macromolecules, 1992, 25, 4297-4301.

Une deuxième méthode est la polycondensation ou polyaddition de dérivés des monomères tels que le TVF correctement fonctionnalisés par exemple en incorporant, des bromes en position alpha des thiophènes des extrémités. Il est alors possible d'utiliser les conditions de couplage de Yamamoto, la polymérisation se faisant par polyaddition en présence d'un complexe de nickel "zéro valent". Un tel procédé est décrit par exemple dans Macromolecules, 1999, 32, 4519-4524.

On peut cependant envisager d'autres méthodes de préparation des polymères, notamment par une réaction de copolymérisation entre un dérivé disubstitué du thiénylène ou du vinylidène et un dérivé disubstitué de la fluorénone. Mais d'autres méthodes encore peuvent également être utilisées.

Les composés selon l'invention peuvent être utilisés dans toutes sortes de dispositifs optoélectroniques tels que les cellules photovoltaïques, les transistors à effet de champ et les capteurs électrochimiques.

Sur les figures 2 et 4, on a représenté une cellule photovoltaïque qui comprend une couche active dans laquelle est incorporé un composé selon l'invention mono-, oligo- ou polymère mélangé à un accepteur (les figures 2 et 4 sont respectivement les vues de côté et vue du dessus).

Cette cellule est fabriquée sur un substrat transparent (1) en un matériau qui peut être souple ou rigide, par exemple du verre et sur lequel se trouve déposée une couche conductrice (2) constituée d'oxyde de métaux par exemple d'oxyde d'indium et étain (Indium Tin Oxide ou ITO).

Le film d'ITO (2) est gravé par exemple sur un tiers de la surface. L'échantillon est ensuite nettoyé aux ultrasons dans différentes solutions : de préférence acétone, puis éthanol, il est ensuite rincé à l'eau désionisée, au TDF4 puis il est rincé et séché à l'étuve.

Des contacts Chrome-or (3) sont déposés sous vide pour permettre ensuite la mesure de la caractéristique I(V) de la cellule. Une pré-couche (4) qui peut être de la poly(aniline) à l'état conducteur ou du PEDOT (poly(éthylènedioxy-thiophène)) dopé au PSS (poly styrène sulfonate), est déposée sur ce substrat. L'épaisseur de cette "pré-couche" (4) est généralement comprise entre 10 et 150 nm mais elle est de préférence de 80 nm.

La couche active organique (5) constituée du donneur d'électrons conjugué selon l'invention sous sa forme mono, oligo ou polymère, mélangé à l'accepteur, de préférence le PCBM (1-(3-méthoxycarbonyl)-propyl-1-phényl-[6,6]-C61) qui est un dérivé soluble du fullerène, est déposée par voie liquide directement sur cette "pré-couche" décrite précédemment.

En ce qui concerne l'accepteur utilisé dans la couche active, il est à noter que le PCBM peut être remplacé par n'importe quel autre accepteur de type organique présentant des niveaux énergétiques équivalents tels que les dérivés du C₆₀, ainsi que les dérivés du C₇₀, les nanotubes de carbone, le pérylène, le tétracyanoquinodiméthane (TCNQ) ou les quinoxalines. Le PCBM peut également être remplacé par n'importe quel accepteur inorganique présentant les mêmes caractéristiques, tels que les nanocristaux de semiconducteurs, enrobés ou non par une couche organique.

Pour le dépôt de la couche active (5), le donneur et l'accepteur sont en solution dans un solvant ou une combinaison de solvants organiques.

Ces solvants peuvent être aliphatiques, aromatiques, ou hétéroaromatiques, substitués ou non. Plus particulièrement, ces solvants sont aromatiques, apolaires, et peuvent être choisis par exemple parmi le toluène, l'ortho-dichlorobenzène, le chlorobenzène et leurs mélanges. Le chlorobenzène est le solvant préféré.

La concentration de l'accepteur est généralement comprise entre 0,1 et 100 g/L et elle est de préférence de 10 g/L et la concentration du donneur est comprise généralement entre 0,1 et 100 g/L et elle est de préférence de 8,75 g/L.

L'électrode est constituée d'un film généralement de LiF (6) d'une épaisseur généralement de 0,5 à 5 nm, de préférence de 1,3 nm, déposée par exemple sous vide sur la couche active (5) et d'une couche par exemple d'aluminium déposée par exemple sous vide de 5 à 200 nm, mais de préférence 70 nm qui vient recouvrir ce film de LiF.

Sur cette couche par exemple d'Al est définie une surface éclairée (7) généralement circulaire d'un diamètre par exemple de 6 mm.

La cellule photovoltaïque a généralement la forme d'un rectangle avec par exemple une largeur de 17 mm et une longueur de 25 mm.

L'invention va maintenant être décrite en référence aux exemples suivants, donnés à titre illustratif et non limitatif.

### EXEMPLE 1

Dans cet exemple, on réalise la synthèse du monomère TVF (à savoir 2,7-bis(5-[(E)-1,2-bis(3-octylthièn-2-yl)éthylène])-fluorén-9-one) selon l'invention.

### Réactifs et produits chimiques

Tous les réactifs et les produits chimiques ont été acquis auprès de Aldrich.

Le THF a été distillé sur du sodium-benzophénone avant utilisation.

Les autres réactifs et produits chimiques ont été utilisés tels que reçus.

### Techniques de caractérisation

La chromatographie en couche mince a été réalisée sur des lames de chromatographie en couche mince gel de silice sur particule d'aluminium, taille 2 à 25 µm, taille de pore 60 ^{°}*A*. La silice utilisée pour la chromatographie flash est la Merck^{®}60 (70-230 mesh). Toutes les molécules synthétisées ont été caractérisées par RMN du ¹H et du ¹³C et analyse élémentaire.

Les spectres de RMN ont été enregistrés sur un spectromètre BRUCKER^{®}AC 200 MHz ou Varian^{®} 400 MHz. Du chloroforme-d, de l'acétone-d6 ou du DMSO-d6 contenant du TMS en tant qu'étalon interne ont été utilisés en tant que solvant en fonction de la solubilité du matériau.

Les analyses élémentaires ont été réalisées par le service d'analyse du CNRS de Vernaison (France).

### Synthèse des précurseurs

Mode opératoire général pour la préparation du précurseur dioxaborinane :

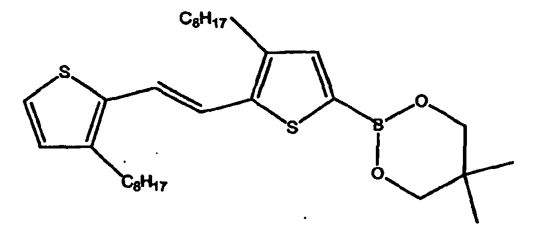

### 5,5-diméthyl-2-[E-(5-(3-octylthiène-2-yl-vinyl)-(3-octylthièn-2-yl)][1,3,2]dioxoborinane

1,46 mL (2,33 mmol) de n-butyllithium sous la forme d'une solution 1,6 M dans l'hexane ont été ajoutés goutte à goutte, à -50°C pendant 5 minutes, à une solution agitée de 884 mg (2,12 mmol) de (E)-1,2-bis(3-octylthièn-2-yl) éthylène dans le THF sec (15 mL).

Ce mélange a été encore agité pendant 60 minutes à -50°C puis refroidi jusqu'à -78°C. A ce stade, 1,72 mL (6,38 mmol) de tributylborate ont été ajoutés rapidement dans le réacteur, et on a laissé la solution se réchauffer jusqu'à la température ambiante.

Lors du réchauffement, le mélange réactif est devenu laiteux. Il a alors été versé dans de l'HCl 1 M contenant de la glace (40 mL) puis extrait avec de l'étheréthylique. La phase organique a été lavée 2 fois avec de la saumure et séchée sur du sulfate de magnésium en présence de 5,20 g (50 mmol) de néopentyl glycol pendant 30 minutes. La séparation ultérieure de l'agent dessicant par filtration et l'élimination du solvant en utilisant un évaporateur rotatif ont donné une huile orange.

Une purification plus poussée par une chromatographie sur colonne de gel de silice avec un mélange hexane-éther (9:1) en tant qu'éluant a donné le 5,5-diméthyl-2-[E-(5-(3-octylthiène-2-yl-vinyl)-(3-octylthièn-2-yl)][1,3,2]dioxoborinane. Huile orange visqueuse (rendement : 61%).

¹H-NMR (CDCl₃, 200 MHz, ppm) : δ : 7,24 (s, 1H), 7,05 (d, 1H, *J*=15,6 Hz), 7,00 (d, 1H, *J*=4,8 Hz), 6,90 (d, 1H, *J*=15,8 Hz.), 6,76 (d, 1H, *J*=5,4 Hz), 3,69 (s, 4H, 2,56 (t, 4H, *J*=7,26 Hz), 1,45-1,60 (m, 4H), 1,15-1,28 (m, 20H), 0,96 (s, 6H), 0,75-0,85 (m, 6H)). ¹³C-NMR (CDCl₃) 200 MHz, ppm). δ 142,28, 141, 89, 141, 16, 138, 35, 136,26, 129, 83, 122, 70, 120, 54, 119, 34, 72,43 (2O*C*H₂), 32,05 (2C), 31,86 (2C), 30,97, 30,86, 29,68 (Cq), 29,42, 29,68, 29,24, 29,21, 28,47, 28,31, 22,65 (2CH₃), 21,87 (2C), 14,06 (2C). C-bore non observée. IR (KRr, cm⁻¹) : 3016 (w), 2956 (s), 2922 (s), 2854 (s), 1520 (w), 1536 (w), 1476 (m), 1458 (m), 1416 (m), 1368 (m), 1380 (m), 1278 (s), 1268 (s), 1298 (s), 1250 (s), 1182 (w), 1108 (m), 1020 (w), 932 (w), 914 (s), 806 (w), 742 (s), 680 (w), 646 (m). Analyse élémentaire : calculée pour C₃₁H₄₉BO₂S₂ : C, 70,43% ; H, 9,34% ; S, 12,13%. Trouvée : C, 70,79% ; H, 9,66% ; S, 12,24%.

### Mode opératoire général pour la préparation du précurseur à l'étain

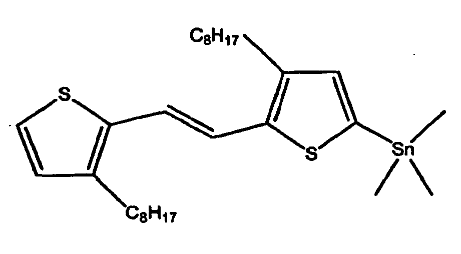

### [E(5-(3-octylthiène-2-yl-vinyl)-(3-octylthièn-2-yl)]-triméthylétain

Une solution de (E)-1,2bis-(3-octylthièn-2-yl)éthylène (1,15 g, 2,76 mmol) dans le THF sec (20 mL) a été refroidie jusqu'à -78°C et 1,9 mL (3,3 mmol, 1,1 éq) d'une solution de nBuLi dans l'hexane (1,6 M) a été lentement ajoutée.

On a laissé la solution se réchauffer jusqu'à -45°C et après 50 minutes sous agitation, la solution rougeâtre a ensuite été refroidie à -78°C. A ce stade, une solution de chlorure de triméthylétain (606 mg, 3,03 mmol, 1,01 éq) dans 4 mL de THF sec a été ajoutée. La solution est devenue immédiatement orange et on l'a alors laissé se réchauffer jusqu'à la température ambiante. Le solvant a été éliminé, puis le résidus a été extrait avec de l'éther diéthylique et lavé deux fois avec de la saumure. La phase organique a été séchée sur du MgSO₄, et après évaporation du solvant, on a récupéré 1,45 g d'une huile orange foncé. L'analyse par RMN¹H de cette huile résiduelle indique qu'il s'agit d'un mélange de produit à l'étain (48%) et de la molécule initiale (811 mg) (rendement 51%). A cause de l'instabilité du groupe stannyle lors de la chromatographie sur gel de silice et du caractère dangereux du produit, ce mélange a été utilisé sans purification plus poussée pour la réaction de couplage de Stille.

### Synthèse du monomère TVF

### 2,7-bis(5-[(E)-1,2-bis(3-octylthièn-2-yl)éthylène])-fluorèn-9-one. TVF

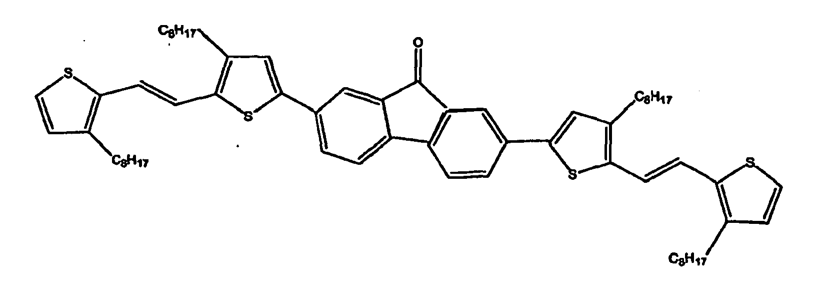

### Synthèse à partir du 5,5-diméthyl(2-[E-(5-(3-octylthièn-2-yl-vinyl)-(3-octylthièn-2-yl)][1,3,2]dioxoborinane (couplage de Suzuki)

183 mg de 2,7-dibromo-fluorèn-9-one, 629 mg de 5,5-diméthyl-2-[E-(5-3-octylthièn-2-yl-vinyl)-(3-octylthièn-2-yl)][1,3,2]dioxoborinane, 280 mg de K₃PO₄, 50 mg de Pd (P(Ph)₃)₄ et 12 mL de DMF ont été utilisés.

Le mélange a été chauffé à 90°C pendant 12 heures. Après un traitement habituel, le produit brut a été purifié par chromatographie sur colonne (gel de silice, pentane-éther, 97:3) pour donner 960 mg (76,4%) d'un produit orange huileux. Après un traitement habituel, le produit brut a été purifié par chromatographie sur colonne (gel de silice, pentane-éther, 97:3) puis par une seconde chromatographie sur colonne en utilisant l'hexane-CHCl₃ (90:10) en tant qu'éluant, pour donner 81 mg (15%) d'un produit orange foncé cireux.

### Synthèse à partir du [E-5(3-octylthièn-2-yl-vinyl)-(3-octylthièn-2-yl)]-triméthylétain (couplage de Stille).

215 mg de 2,7-dibromo-fluorèn-9-one (0,63 mmol) et le mélange notamment le composé à l'étain (1,40 mmol) ont été placés dans le DMF anhydre (8 mL). Le mélange a été agité sous argon pendant 10 minutes, puis 297 mg de K₃PO₄ (1,40 mmol) et 52 mg de Pd (P(Ph)₃)₄ (0,044 mmol) dans 8 mL de DMF ont été ajoutés. Le mélange a été maintenu pendant une durée supplémentaire de 15 heures à 95°C avec agitation constante puis on l'a laissé se refroidir jusqu'à la température ambiante. Après un traitement habituel, le produit brut a été purifié par chromatographie sur colonne (gel de silice, pentane-éther, 95:5) pour donner 600 mg (93%) d'un produit cireux rouge foncé.

¹H-NMR (CDCl₃, 200 MHz, ppm) : δ : 7,75 (d, 2H, *J*=1,34 Hz), 7,56 (dd, 2H, *J*=7,80 et 1,74 Hz), 7,34 (d, 2H, *J*=7,80 Hz), 7,05 (s, 2H), 7,01 (d, 2H, *J*=5,24 Hz), 6,90 (d, 4H, *J*=4,70 Hz), 6,78 (d, 2H, *J*=5,11 Hz), 2,59 (m, 8H, *J*=8,74 Hz), 1,60-1,48 (m, 8H), 1,30-1,18 (m, 40 H), 0,84-0,77 (m, 12H). ¹³C-NMR (CDCl₃, 200 MHz, ppm) : δ : 193,30 (C=O), 142,52 (2C), 142,07 (2C), 141,14 (2C), 139,28 (2C), 136,86 (2C), 136,86 (2C), 136,18 (2C), 135,08 (2C), 131,00 (2C), 129,88 (2C), 127,80 (2C), 126,38 (2C), 122,84 (2C), 120,95 (2C), 120,63 (2C), 119,82 (2C), 118,85 (2C), 31,89 (4C), 30,94 (4C), 30,78 (4C), 29,39 (4C), 29,25 (4C), 28,48 (4C), 22, 66 (4C), 14,08 (4C). IR (KBr, cm⁻¹) : 3066 (w), 3016 (m), 2954 (s), 2924 (s), 2852 (s), 1720 (s), 1600 (w), 1584 (w), 1542 (w), 1472 (s), 1464 (s), 1456 (s), 1436 (s), 1378 (w), 1294 (m), 1260 (m), 1086 (m), 1024 (m), 928 (s), 906 (m), 820 (s), 784 (s), 722 (m), 680 (w), 654 (w). Analyse élémentaire calculée pour C₆₅H₈₉OS₄ : C, 77,33% ; H, 8,39% ; S, 12,70%. Trouvée : C, 76,70% ; H, 8,42% ; S 12,70%.

### Exemple 2 : synthèse du polymère PTVF

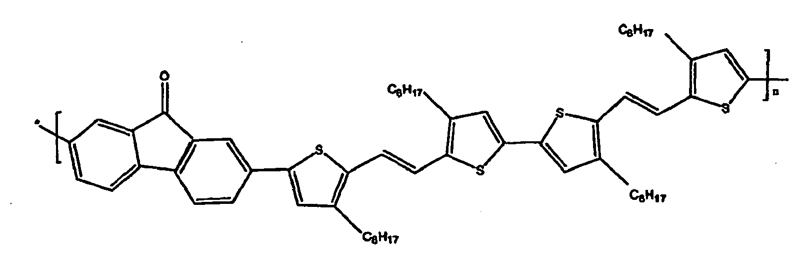

Une solution de 260 mg de chlorure ferrique anhydre dans un mélange de solvants constitué de 5 mL de nitrométhane et 5 mL de chloroforme a été ajouté goutte à goutte à une solution du co-monomère (405 mg) dans 15 mL de chloroforme fraîchement distillé et dégazé.

L'addition a été réalisée à 0°C avec agitation constante pendant une durée de 90 minutes. A la fin de l'addition, le mélange a été réchauffé jusqu'à 10°C et a été maintenu à cette température pendant encore 60 minutes.

On a laissé alors le mélange réactionnel se réchauffer jusqu'à la température ambiante et on l'a agité pendant 12 heures.

Ensuite, il a été concentré par aspiration sous vide puis précipité dans 100 mL de méthanol. Le polymère brut a ensuite été dissout dans 50 mL de chloroforme et lavé quatre fois avec une solution aqueuse à 0,1 M d'ammoniac (150 mL chaque fois). Dans l'étape suivante, le polymère a été agité pendant 48 heures avec la même solution aqueuse.

Tel que synthétisé, le polymère contient habituellement de petites quantités de dopants de matrice chimique non identifiée et requiert et encore un traitement d'élimination des dopants.

L'élimination des dopants a été réalisée par lavage de la solution du polymère dans le chloroforme avec une solution aqueuse d'EDTA (0,05 M, 200 mL).

Le polymère a ensuite été lavé deux fois avec de l'eau puis séché sous vide.

### Exemple 3 : réalisation de la cellule

La cellule utilisée pour des essais a la configuration représentée sur la figure 2 et la figure 4. Elle comporte un substrat en verre, recouvert d'une couche d'ITO qui est elle-même recouverte de deux couches de PEDOT/PSS (polymère conducteur) (produit commercialisé par BAYER).

La couche supérieure de PEDOT/PSS est recouverte d'une couche active organique du monomère, oligomère ou polymère selon l'invention (par exemple TVF ou PTVF) en mélange avec du PCBM qui est un dérivé soluble du fullerène.

La couche active organique est revêtue enfin successivement d'une couche de LiF et d'une couche d'aluminium.

La configuration de la cellule est donc la suivante :

Substrat de verre/ITO/PEDOT-PSS/oligomère ou polymère + PCBM/LiF-Al

La cellule est préparée de la manière suivante :
La surface des substrats est de 4,25 cm².
Conditions de dépôt par enduction centrifuge (ou tournette) :
   Dans la première étape la durée de l'enduction est 40 s à 1500 tr/min avec une accélération qui permet d'atteindre les 1500 tours en 4 s puis dans la deuxième étape la durée de l'enduction est de 20 s à 2000 tr/min, avec une accélération de 4 s pour passer de 1500 tours à 2000 tours.

Etape 1 : deux couches successives de PEDOT/PSS (produit commercialisé par BAYER) sont déposées à la tournette sur le substrat de verre recouvert d'ITO. L'épaisseur de la couche obtenue est de 80 nm. Le dépôt est réalisé à l'air, puis séché sous vide.
Etape 2 : la couche active : mélange PCBM (dérivé soluble du fullerène) et TVF ou mélange PCBM et PTVF, est déposée à la tournette.
   Les dépôts sont réalisés à partir d'une solution contenant : 10 mg de PCBM et 8,75 mg de TVF ou de PTVF dans 1 mL de chlorobenzène. Le dépôt est réalisé sous azote en boîte à gants. La surface active est de 2 cm².
Etape 3 : une couche de LiF (1,3 nm) est déposée sous vide puis une couche d'aluminium (70 nm) est déposée sous vide. La surface est environ de 0,3 cm².

### Exemple 4 (comparatif)

Dans cet exemple, on réalise une cellule de la même manière que dans l'exemple 3 à la seule différence que la couche active est constituée d'un mélange de PCBM (accepteur) et de MDMO-PPV selon le document WO-A1-03/078498 au lieu d'un mélange de PCBM et de TVF ou de PTVF.

### Exemple 5 : caractérisation des cellules

La cellule selon l'invention préparée dans l'exemple 3 est alors caractérisée en boîte à gants sous atmosphère contrôlée, à savoir une atmosphère d'un azote avec taux d'oxygène et de vapeur d'eau inférieurs à 1 ppm et à température ambiante. Les caractéristiques courant-tension (I(V)) sont enregistrées sous éclairement AM1,5 à une puissance de 80 W/m².

La cellule comparative comprenant une couche active selon l'art antérieur préparée dans l'exemple 4 est caractérisée de la même manière.

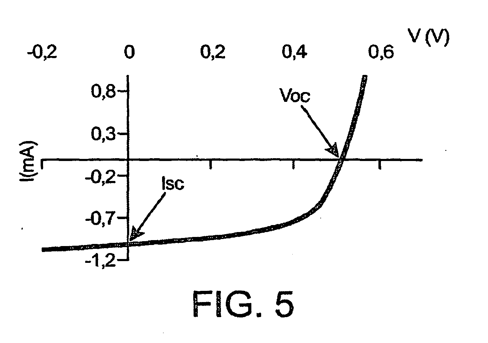

Le Tableau 1 suivant comporte les caractéristiques des cellules avec des couches actives selon l'invention réalisées à partir du monomère TVF et du polymère correspondant PTVF, et des couches actives non-conformes à l'invention avec un mélange PCBM/MDMO-PPV. Les résultats de ces essais et d'autres sont présentés sur les figures 1, 3 et 5.

**Tableau 1**

| | **MDMO-PPV** | **TVF** | **PTVF** |
|---|---|---|---|
| Isc (mA) | 1,42 | 0,76 | 1,01 |
| JsC (mA/cm²) | 3,93 | 2,39 | 3,06 |
| Voc (V) | 0,70 | 0,70 | 0,50 |
| Pmax | 1,31 | 0,17 | 0,88 |
| (mW/cm²) | | | |
| FF | 0,43 | 0,32 | 0,58 |
| Rdt (%) | 1,47 | 0,66 | 1,10 |

| | | | |
|---|---|---|---|
| Isc en mA correspond au courant de court circuit. Jsc en mA/cm² correspond à la densité de courant en court circuit. Voc en V correspond à la tension de circuit ouvert. Pmax correspond à la densité de puissance maximale fournie par la cellule (mW/cm²). FF correspond au Fill Factor (facteur correctif du rendement de la cellule) : idéalement FF=1. Le rendement correspond au taux de conversion des photons en électrons. | | | |

Les performances des cellules présentées dans le Tableau 1 démontrent l'amélioration de certaines caractéristiques des cellules comportant la couche active selon l'invention par rapport aux cellules avec une couche active comportant du MDMO-PPV (WO 03/078498 A1) et testées dans les mêmes conditions que les composés de l'invention.

Notamment, avec le PTVF, la cellule présente un facteur de forme (en anglais : Fill Factor, FF) 1,3 fois plus important.

Le pouvoir rectifiant de la cellule est meilleur dans le cas de PTVF que pour MDMO-PPV.

Le monomère TVF quant à lui présente une tension de circuit ouvert (Voc) égale à celle déterminée pour le polymère MDMO-PPV.

## Revendications

1. Composé monomère, oligomère, ou polymère répondant à la formule (I) suivante : 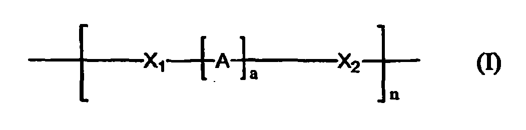 dans laquelle
- A représente un groupe polycyclique éventuellement substitué par un ou plusieurs groupements R identiques ou différents choisis parmi les radicaux alkyle, ledit groupe polycyclique comprenant au moins deux cycles choisis parmi les cycles aromatiques carbonés et/ou les hétérocycles aromatiques, et comprenant au moins un groupe susceptible de permettre le rattachement d'un groupe chromophore,
- X₁ et X₂ identiques ou différents, représentent chacun indépendamment un groupe de formule (II) : 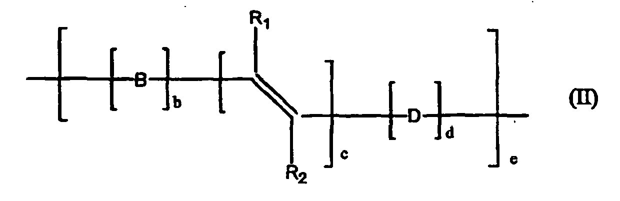 dans laquelle
• B et D, identiques ou différents représentent chacun indépendamment un cycle aromatique carboné ou un hétérocycle aromatique éventuellement substitué par un ou plusieurs groupements R ;
• R₁ et R₂, identiques ou différents, représentent chacun indépendamment un groupement choisi parmi l'atome d'hydrogène, les groupements R, les groupements cyano, nitro, les atomes d'halogène et les atomes de deutérium ;
• b et d sont des nombres entiers de 1 à 100, de préférence de 1 à 12 ;
• c est un nombre entier de 1 à 20, de préférence de 1 à 5 ;
• est un nombre entier de 1 à 100, de préférence de 1 à 10 ;
• X₁ et/ou X₂ sont éventuellement substitués par un ou plusieurs groupes de formule (II) identiques ou différents du groupe X₁ ou X₂ substitué ;
- n est un nombre entier de 1 à 1000, de préférence de 1 à 200 ;
- a est un nombre entier de 1 à 100, de préférence de 1 à 5 ;
- un ou plusieurs des atomes d'hydrogène de la formule (I) peuvent être remplacés par un atome de deutérium ou de fluor.

2. Composé selon la revendication 1, dans lequel ledit groupe susceptible de permettre le rattachement d'un groupe chromophore est un groupe 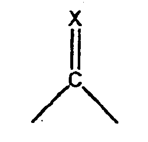 où X représente O, S, Se ou N-R₃, R₃ étant choisi parmi les groupes issus de la réaction d'un groupe carbonyle (X=O) avec une amine pour former une fonction imine.

3. Composé selon l'une quelconque des revendications précédentes dans lequel, dans la formule (I) le groupe polycyclique du groupe A est un groupe polycyclique condensé.

4. Composé selon la revendication 1, dans lequel dans la formule (I), le groupe A est choisi parmi les groupements fluorènone, truxénone, indénofluorénone, benzofluorénone, dibenzofluorénone, indénofluorénedione, cyclopentafluorénone, cyclopentafluorénedione, thiopyranone, phénanthrénone, cyclopentadithiophénone, et les groupements dérivés de ceux-ci ; les groupements dérivés de la xanthone de formule 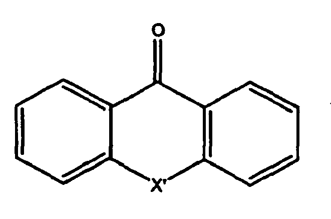 où X' représente un radical -CO-, -S-, -Se-, -NH-, -CH₂-, -CH₂=CH₂-, les groupements dérivés de la 4-pipéridone comprenant au moins deux cycles aromatiques carbonés et/ou hétérocycles aromatiques condensés, ces groupements étant éventuellement substitués par un ou plusieurs groupements R et/ou éventuellement condensés avec un ou plusieurs cycles aromatiques carbonés et/ou hétérocycles aromatiques.

5. Composé selon la revendication 1, dans lequel, dans la formule (II), B et D représentent un thiophène, de préférence un groupe thièn-2,5-diyle.

6. Composé selon l'une quelconque des revendications précédentes, dans lequel B et D, en particulier lorsqu'ils représentent un thiophène, sont substitués par un radical alkyle de 1 à 10C, tel que le radical n-octyle.

7. Composé selon la revendication 1 qui est un monomère (n=1) symétrique.

8. Composé selon la revendication 1, qui est un oligomère ou polymère, contenant de préférence un seul type de groupe A, alterné, régiorégulier, sans défaut de couplage.

9. Composé selon la revendication 1, dans lequel le groupe A répond à la formule suivante : 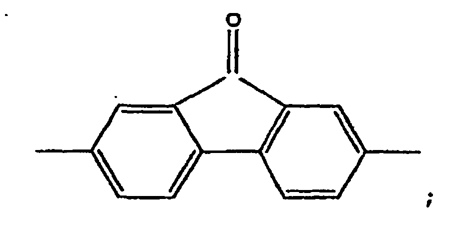 et X₁ et X₂, identiques ou différents répondent à la formule suivante : 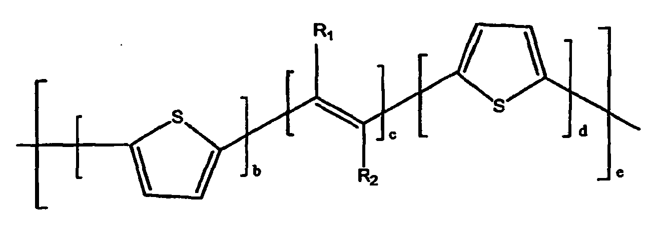 le groupe A et les groupes thiényle pouvant être éventuellement substitués par un ou plusieurs groupements R.

10. Composé selon la revendication 1, répondant à la formule suivante : 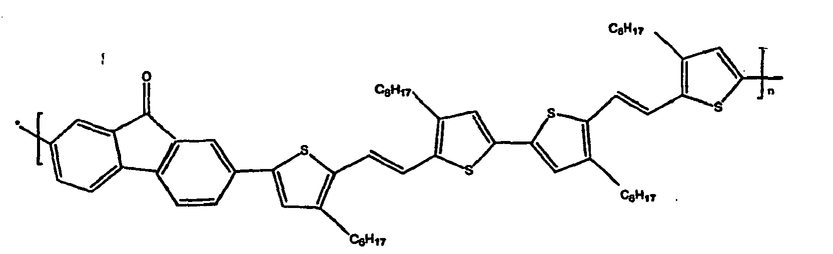 qui est le 2,7-bis(5-[(E)-1,2-bis(3-octylthièn-2-yl)éthylène])-fluorèn-9-one (TVF) si n=1 ; ou le poly(2,7-bis(5-[(E)-1,2-bis(3-octylthièn-2-yl)éthylène])-fluorèn-9-one)) (poly(TVF)) si n est supérieur à 1.

11. Utilisation du composé selon l'une quelconque des revendications 1 à 10 dans un dispositif optoélectronique.

12. Utilisation selon la revendication 11 dans lequel ledit dispositif optoélectronique est une cellule photovoltaïque, un transistor à effet de champ ou un capteur électrochimique.

13. Cellule photovoltaïque comprenant une couche active comprenant un donneur d'électrons constitué par le composé selon l'une quelconque des revendications 1 à 10 et un accepteur d'électrons.

14. Cellule photovoltaïque selon la revendication 13, dans laquelle ledit accepteur d'électrons est choisi parmi les accepteurs organiques tels que le PCBM (1-(3-méthoxycarbonyl)-propyl-1-phényl-[6,6]-C61), les dérivés du C₆₀, les dérivés du C₇₀, les nanotubes de carbone, le pérylène, le tétracyanoquinodiméthane (TCNQ) et les quinoxalines ; et les accepteurs inorganiques tels que les nanocristaux de semiconducteurs, enrobés ou non par une couche organique.

## Claims

1. Monomeric, oligomeric or polymeric compound corresponding to the following formula (I): 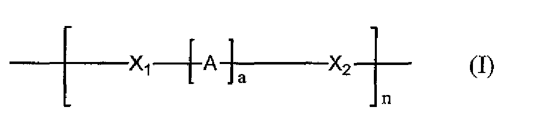 in which
- A represents a polycyclic group optionally substituted by one or more identical or different R groups chosen from alkyl radicals, the said polycyclic group comprising at least two rings chosen from aromatic carbon rings and/or aromatic heterocycles and comprising at least one group capable of making possible the attachment of a chromophoric group,
- X₁ and X₂, which are identical or different, each independently represent a group of formula (II): 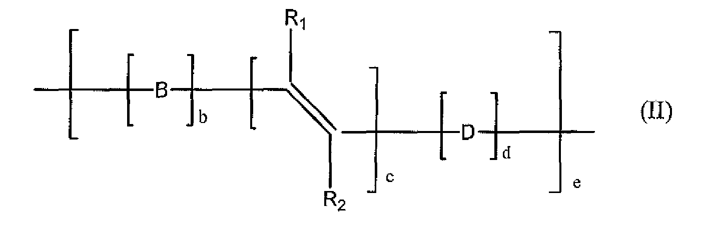 in which
• B and D, which are identical or different, each independently represent an aromatic carbon ring or an aromatic heterocycle optionally substituted by one or more R groups;
• R₁ and R₂, which are identical or different, each independently represent a group chosen from the hydrogen atom, R groups, the cyano group, the nitro group, halogen atoms and deuterium atoms;
• b and d are integers from 1 to 100, preferably from 1 to 12;
• c is an integer from 1 to 20, preferably from 1 to 5;
• e is an integer from 1 to 100, preferably from 1 to 10;
• X₁ and/or X₂ are optionally substituted by one or more groups of formula (II) identical to or different from the substituted X₁ or X₂ group;
- n is an integer from 1 to 1000, preferably from 1 to 200;
- a is an integer from 1 to 100, preferably from 1 to 5;
- one or more of the hydrogen atoms of the formula (I) can be replaced by a deuterium or fluorine atom.

2. Compound according to Claim 1, in which the said group capable of making possible the attachment of a chromophoric group is a group 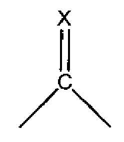 where X represents O, S, Se or N-R₃, R₃ being chosen from the groups resulting from the reaction of a carbonyl group (X=O) with an amine to form an imine functional group.

3. Compound according to either one of the preceding claims, in which, in the formula (I), the polycyclic group of the A group is a condensed polycyclic group.

4. Compound according to Claim 1, in which, in the formula (I), the A group is chosen from fluorenone, truxenone, indenofluorenone, benzofluorenone, dibenzofluorenone, indenofluorenedione, cyclopentafluorenone, cyclopentafluorenedione, thiopyranone, phenanthrenone, cyclopentadithiophenone groups and the groups derived from these; groups derived from the xanthone of formula 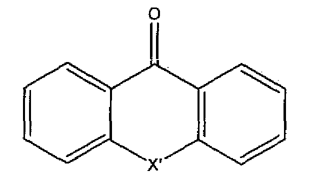 where X' represents a -CO-, -S-, -Se-, -NH-, -CH₂- or -CH₂=CH₂- radical; groups derived from 4-piperidone comprising at least two condensed aromatic carbon rings and/or aromatic heterocycles, these groups optionally being substituted by one or more R groups and/or optionally condensed with one or more aromatic carbon rings and/or aromatic heterocycles.

5. Compound according to Claim 1, in which, in the formula (II), B and D represent a thiophene, preferably a thien-2,5-diyl group.

6. Compound according to any one of the preceding claims, in which B and D, in particular when they represent a thiophene, are substituted by an alkyl radical of 1 to 10 carbon atoms, such as the n-octyl radical.

7. Compound according to Claim 1, which is a symmetrical monomer (n=1).

8. Compound according to Claim 1, which is a regioregular alternating oligomer or polymer which preferably comprises a single type of A group and which is devoid of coupling defects.

9. Compound according to Claim 1, in which the A group corresponds to the following formula: 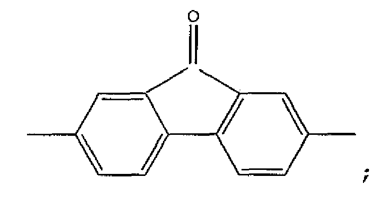 and X₁ and X₂, which are identical or different, correspond to the following formula: 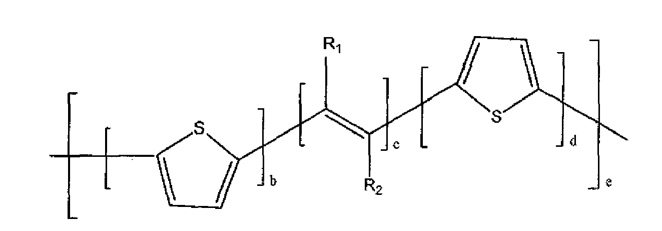 it being possible for the A group and the thienyl groups optionally to be substituted by one or more R groups.

10. Compound according to Claim 1, corresponding to the following formula: 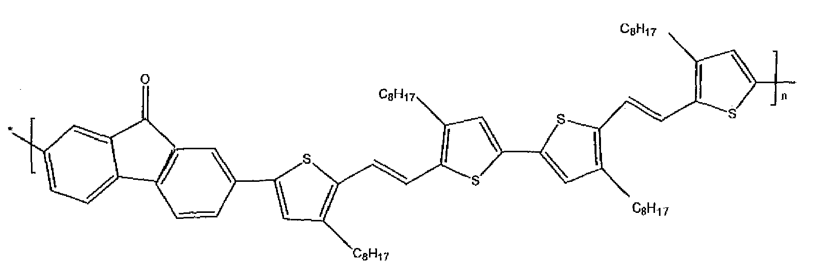 which is 2,7-bis(5-[(E)-1,2-bis(3-octylthien-2-yl)ethylene])-fluoren-9-one (TVF) if n=1; or poly(2,7-bis(5-[(E)-1,2-bis(3-octylthien-2-yl) ethylene]) -fluoren-9-one) (poly(TVF)) if n is greater than 1.

11. Use of the compound according to any one of Claims 1 to 10 in an optoelectronic device.

12. Use according to Claim 11, in which the said optoelectronic device is a photovoltaic cell, a field-effect transistor or an electrochemical sensor.

13. Photovoltaic cell, comprising an active layer comprising an electron donor composed of the compound according to any one of Claims 1 to 10 and an electron acceptor.

14. Photovoltaic cell according to Claim 13, in which the said electron acceptor is chosen from organic acceptors, such as PCBM (1-(3-methoxycarbonyl)propyl-1-phenyl-[6,6]-C61), C₆₀ derivatives, C₇₀ derivatives, carbon nanotubes, perylene, tetracyanoquinodimethane (TCNQ) and quinoxalines; and inorganic acceptors, such as semiconductor nanocrystals, which may or may not be coated by an organic layer.

## Patentansprüche

1. Monomere, oligomere oder polymere Verbindung gemäß der folgenden Formel (I): 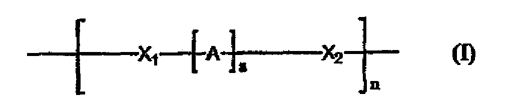 worin
- A eine polycyclische Gruppe darstellt, die gegebenenfalls durch eine oder mehrere, identische oder verschiedene, aus Alkylresten ausgewählte Gruppen R substituiert ist, wobei die polycyclische Gruppe wenigstens zwei Cyclen umfasst, die aus aromatischen Kohlenstoffcyclen und/oder aromatischen Heterocyclen ausgewählt sind, und wenigstens eine Gruppe umfasst, die das Binden einer chromophoren Gruppe ermöglicht,
- X₁ und X₂, die gleich oder verschieden sind, jeweils unabhängig eine Gruppe der Formel (II) darstellen: 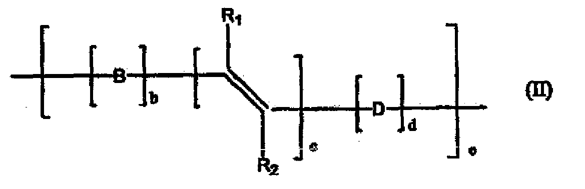 worin
• B und D, die gleich oder verschieden sind, jeweils unabhängig einen gegebenenfalls durch eine oder mehrere Gruppen R substituierten aromatischen Kohlenstoffcyclus oder aromatischen Heterocyclus darstellen,
• R₁ und R₂, die gleich oder verschieden sind, jeweils unabhängig eine Gruppe darstellen, die aus einem Wasserstoffatom, R-Gruppen, Cyan- und Nitrogruppen, Halogenatomen und Deuteriumatomen ausgewählt sind,
• b und d ganze Zahlen von 1 bis 100, vorzugsweise 1 bis 12 sind,
• c eine ganze Zahl von 1 bis 20, vorzugsweise 1 bis 5 ist,
• e eine ganze Zahl von 1 bis 100, vorzugsweise 1 bis 10 ist,
• X₁ und/oder X₂ gegebenenfalls durch eine oder mehrere, mit der substituierten Gruppe X₁ oder X₂ identische oder dazu verschiedene Gruppen der Formel (II) substituiert sind,
- n eine ganze Zahl von 1 bis 1000, vorzugsweise 1 bis 200 ist,
- a eine ganze Zahl von 1 bis 100, vorzugsweise 1 bis 5 ist,
- und ein oder mehrere Wasserstoffatome der Formel (I) durch ein Deuterium- oder Fluoratom ersetzt sein können.

2. Verbindung gemäß Anspruch 1, bei der die Gruppe, die das Binden einer chromophoren Gruppe ermöglicht, eine Gruppe 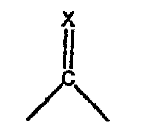 ist, worin X, O, S, Se oder N-R₃ bedeutet, wobei R₃ aus den Gruppen ausgewählt ist, die aus der Reaktion einer Carbonylgruppe (X = O) mit einem Amin unter Bilden einer Iminfunktion hervorgehen.

3. Verbindung gemäß einem der vorangehenden Ansprüche, bei der in Formel (I) die polycyclische Gruppe der Gruppe A eine kondensierte polycyclische Gruppe ist.

4. Verbindung gemäß Anspruch 1, bei der in Formel (I) die Gruppe A aus Fluorenon--, Truxenon-, Indenofluorenon-, Benzofluorenon-, Dibenzofluorenon-, Indenofluorendion-, Cyclopentafluorenon-, Cyclopentafluorenondion-, Thiopyranon-, Phenanthrenon- und Cyclopentadithiophenongruppen und den davon abgeleiteten Gruppen; von dem Xanthon der Formel 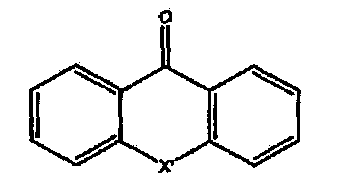 worin X' einen Rest -CO-, -S-, -Se-, -NH-, -CH₂-, -CH₂=CH₂- darstellt, abgeleiteten Gruppen und den Gruppen ausgewählt ist, die von 4-Piperidon abgeleitet sind, das wenigstens zwei kondensierte, aromatische Kohlenstoffcyclen und/oder aromatische Heterocyclen umfasst, wobei diese Gruppen gegebenenfalls durch eine oder mehrere Gruppen R substituiert sind und/oder gegebenenfalls mit einem oder mehreren aromatischen Kohlenstoffcyclen und/oder aromatischen Heterocyclen kondensiert sind.

5. Verbindung gemäß Anspruch 1, bei der in Formel (II) B und D ein Thiophen, vorzugsweise eine Thien-2,5-diylgruppe darstellen.

6. Verbindung gemäß einem der vorangehenden Ansprüche, bei der B und D, insbesondere wenn sie ein Thiophen bedeuten, durch einen C₁₋₁₀-Alkylrest wie etwa dem Octylrest substituiert sind.

7. Verbindung gemäß Anspruch 1, die ein symmetrisches Monomer (n = 1) ist.

8. Verbindung gemäß Anspruch 1, die ein regioreguläres, alternierendes Oligomer oder Polymer ohne Kupplungsfehler ist, das vorzugsweise einen einzigen Typ der Gruppe A enthält.

9. Verbindung gemäß Anspruch 1, bei der die Gruppe A der folgenden Formel entspricht: 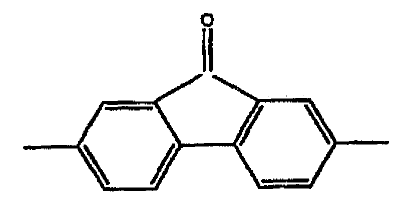 und X₁ und X₂, die gleich oder verschieden sind, der folgenden Formel entsprechen: 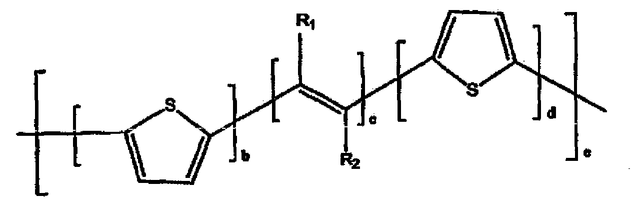 wobei die Gruppe A und die Thienylgruppen gegebenenfalls durch eine oder mehrere Gruppen R substituiert sein können.

10. Verbindung gemäß Anspruch 1, die der folgenden Formel entspricht: 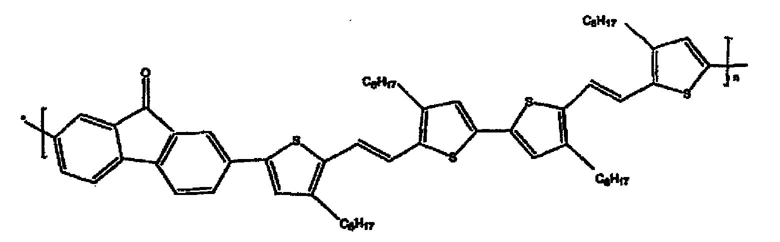 und 2,7-Bis(5-[(E)-1,2-bis(3-octylthien-2-yl)ethylen])-fluoren-9-on (TVF) ist, wenn n = 1, oder Poly(2,7-bis(5-[(E)-1,2-bis(3-octylthien-2-yl)ethylen])-fluoren-9-on) (Poly(TVF)) ist, wenn n größer als 1 ist.

11. Verwendung einer Verbindung gemäß einem der Ansprüche 1 bis 10 in einer optoelektronischen Vorrichtung.

12. Verwendung gemäß Anspruch 11, bei der die optoelektronische Vorrichtung eine Photovoltaikzelle, ein Feldeffekttransistor oder ein elektrochemischer Sensor ist.

13. Photovoltaikzelle umfassend eine aktive Schicht, die einen Elektronendonor, der sich aus der Verbindung gemäß einem der Ansprüche 1 bis 10 besteht und einem Elektronenakzeptor umfasst.

14. Photovoltaikzelle gemäß Anspruch 13, bei der der Elektronenakzeptor aus organischen Akzeptoren wie etwa PCBM (1-(3-Methoxycarbonyl)propyl-1-phenyl-[6,6]-C61), C₆₀-Derivaten, C₇₀-Derivaten, Kohlenstoffnanoröhren, Perylen, Tetracyanchinodimethan (TCNQ) und Chinoxalinen und anorganischen Akzeptoren wie etwa mit einer organischen Schicht überzogenen oder nicht überzogenen Halbleiternanokristallen ausgewählt ist.
